# EUROPÄISCHE PATENTANMELDUNG

(11) **EP 0 952 213 A2**
(43) Veröffentlichungstag der Anmeldung: **27.10.1999**
(21) Anmeldenummer: 99105128.5
(22) Anmeldetag: 26.03.1999
(51) Int. Cl.: C12N 5/06, C12N 5/08, A61K 35/12

(54) **Verfahren zur Induktion einer durch NK-Zellen vermittelten Immunantwort**

(30) Priorität: 27.03.1998 DE 19813759
(71) Anmelder: GSF-Forschungszentrum für Umwelt und Gesundheit, GmbH, 85764 Oberschleissheim (DE)
(72) Erfinder: Multhoff, Gabriele, Dr., 81675 München (DE); Botzler, Claus, Dr., 80796 München (DE)
(74) Vertreter: Reinhard - Skuhra - Weise & Partner

(57) **Zusammenfassung**

Erfindungsgemäß wird ein Verfahren zur Induktion einer durch NK-Zellen vermittelten Immunantwort, ex vivo, bereitgestellt, dadurch gekennzeichnet, daß eine zumindest Tumorzellen oder durch Viren, Bakterien und/oder Pilze infizierte tierische oder menschliche Zellen (Zeilzellen) und NK-Zellen enthaltende physiologische Zellsuspension mit den nachfolgenden Verfahrensschritten in der angegebenen Reihenfolge behandelt wird, um die Sensitivität der Zielzellen für die Lyse durch NK-Zellen zu erhöhen:
a) Hitzebehandeln der in der Suspension enthaltenen Zellen bei einer Temperatur von 38°C bis 43°C für eine Zeit von mindestens 1 Stunde;
b) Absenken der Temperatur auf physiologische Zelltemperatur (etwa 37°C), um den in der Suspension enthaltenen Zellen eine Erholungsphase von mindestens 1 Stunde zu gewähren;
c) Zugabe einer den membrangebundenen Hsp70-Anteil der Zielzellen erhöhenden Verbindung in einer für die Zellen subletalen Konzentration und Einwirkenlassen dieser Verbindung für mindestens 30 Minuten;
d) Erholungsphase von mindestens etwa 1 Stunde bei 37°C.

## Beschreibung

Die vorliegende Erfindung betrifft ein ex-vivo-Verfahren zur Induktion einer durch NK-Zellen vermittelten Immunantwort, eine Verwendung des durch das Verfahren erhaltenen Produkts sowie eine Verwendung des Verfahrens im therapeutischen Bereich.

Etherlipide wie 1-Octadecyl-2-methyl-rac-glycero-3-phosphocholin (ET-18-OCH3, Edelfosin) gehören zur Gruppe von Alkyl-Lysophospholipiden. Alkyl-Lysophospholipide sind synthetische Ansloga des natürlich vorkommenden 2-Lysophosphatidylcholins. Es ist bekannt, daß Alkyl-Lysophospholipide zytotoxisch wirken und selektiv neoplastische Zellen abtöten. Es ist bekannt, daß bei zytotoxischen Konzentrationen von Alkyl-Lysophospolipiden das Tumorzellwachstum in vitro und in vivo durch eine direkte Zerstörung der Tumorzellen gehemmt wird.

Obwohl der Mechanismus der durch Etherlipide induzierten Zytotoxizität unbekannt ist, nimmt man an, daß die Plasmamembran das Hauptziel der zytotoxischen Aktivität von Alkyl-Lysophospholipiden, z.B. von ET-18-OCH3, ist. So wurde beispielsweise gezeigt, daß durch Alkyl-Lysophospholipid-Derivate die Membranpermeabilität, die Membranfluidität, die Lipidzusammensetzung der Membran und ihr Cholesteringehalt beeinflußt werden. Weiterhin wurde ET-18-OCH3 als "Reinigungsmittel" (Purging) in präklinischen Modellen der autologen, hämatopoetischen Stammzelltransplantation und in klinischen Studien der Phasen I/II für das ex vivo-Purging eingesetzt.

Es hat sich jedoch gezeigt, daß z.B. einige leukämische Zellinien, beispielsweise K562, gegen durch ET-18-OCH3 vemittelte zytotoxische Effekte relativ widerstandsfähig sind (1). Eine Purging-Behandlung könnte deshalb mit einem erhöhten Risiko für einen erneuten Ausbruch der Leukämieerkrankung einhergehen, was auf die Reinfusion hochresistenter, leukämischer Zellen in den Patienten zurückzuführen ist. Um diesen Nachteil zu vermeiden und um die zytotoxischen Effekte von ET-18-OCH3 zu erhöhen, wurden weitere zusätzliche Behandlungsverfahren vorgeschlagen, beispielsweise eine in vitro durchzuführende Hyperthermie-Behandlung ((2)-(4)). So offenbart zum Beispiel die Veröffentlichung (2) die Behandlung von HL60-, K562- und KG-1-Leukämiezellen aus dem Knochenmark von Leukämiepatienten mit 50 µg/ml ET-18-OCH3 und einer nachfolgenden Hitzeschockbehandlung bei 42°C für eine Stunde. Hierdurch wird ein direkter zytotoxischer Effekt auf die Tumorzellen erreicht.

Aus (3) ist die Behandlung von BG-1-Ovarial-Karzinomzellen mit 1, 2 bzw. 4 µM ET-18-OCH3 (1 µM entspricht ca. 0,5 µg/ml) für einen Zeitraum von 1 - 12 Tagen und einer nachfolgenden Hitzeschockbehandlung bei 42°C bzw. 44°C bekannt. Dieses Verfahren dient dazu, die Tumorzellen durch direkte, zytotoxische Effekte abzutöten.

Aus (4) ist die Behandlung von BG-1-Zellen mit ET-18-OCH3 bei einer Konzentration von 2 bzw. 8 µM mit einer nachfolgenden Hitzeschockbehandlung bei 42°C für den Zeitraum von 0 - 16 Stunden und von 44°C für einen Zeitraum von 0 - 33 Stunden bekannt. Hierdurch soll ein direkter, verstärkter, zytotoxischer Effekt auf die Tumorzellen erreicht werden.

Aus (5) ist bekannt, daß bei einer nicht-toxischen Behandlung von K562-Zellen mit ET-18-OCH3 eine signifikante Steigerung der Sensitivität von K562-Zellen für die Lysis durch mit Interleukin-2 stimulierte NK-Zellen stattfindet. Weiterhin wird in dieser Literaturstelle ausgeführt, daß ähnliche Effekte durch eine nicht-letale Hitzeschockbehandlung erreichbar seien. Eine Kombination beider Behandlungsverfahren und die zwingende Einhaltung einer bestimmten Reihenfolge bei der Behandlung zur Erreichung der Stimulierung der zytolytischen Aktivität von NK-Zellen für Tumorzellen oder eine synergistische Wirkung wird hierdurch jedoch nicht nahegelegt.

Es ist eine Aufgabe der vorliegenden Erfindung, ein ex vivo-verwendbares Verfahren zur Induktion einer durch NK-Zellen vermittelten Immunantwort bereitzustellen. Ein derartiges Verfahren soll weiterhin so ausgelegt sein, daß die so behandelte Zellsuspension in den Patienten zur Behandlung der Tumorerkrankung reinfundierbar ist.

Diese Aufgabe wird erfindungsgemäß durch das Verfahren gemäß Anspruch 1 der beiliegenden Patentansprüche gelöst. Bevorzugte Ausgestaltungen des Verfahrens ergeben sich aus den Unteransprüchen sowie aus der nachfolgenden Beschreibung mit den Ausführungsbeispielen.

Durch das erfindungsgemäße Verfahren kann eine durch Natürliche Killerzellen vermittelte Immunantwort induziert werden. Hierzu wird eine zumindest Tumorzellen oder durch Viren, Bakterien und/oder Pilze infizierte tierische oder menschliche Zellen, kurz als Zielzellen bezeichnet, und natürliche Killerzellen enthaltende physiologische Zellsuspension mit den nachfolgenden Verfahrensschritten in der angegebenen Reihenfolge behandelt, um die Sensitivität der Zielzellen für die Lysis durch NK-Zellen zu erhöhen:
a) Hitzebehandeln der in der Suspension enthaltenen Zellen bei einer Temperatur von 38°C bis 43°C für eine Zeit von mindestens 1 Stunde;
b) Absenken der Temperatur auf physiologische Zelltemperatur (etwa 37°C ), um den in der Suspension enthaltenen Zellen eine Erholungsphase von mindestens 1 Stunde zu gewähren;
c) Zugabe einer den membrangebundenen Hsp70-Anteil der Zielzellen erhöhenden Verbindung in einer für die Zellen subletalen Konzentration und Einwirkenlassen dieser Verbindung für mindestens 30 Minuten;
d) Erholungsphase von mindestens 1 Stunde bei 37°C.

Erfindungsgemäß wird somit ein Verfahren zur Steigerung der lytischen Wirkung von NK-Zellen für Tumorzellen, insbesondere Leukämiezellen, Lymphomzellen und Zellen aus Metastasen solider Tumore, bereitgestellt. Weiterhin können durch das erfindungsgemäße Verfahren auch Zellen lysiert werden, die durch Viren, Bakterien und/oder Pilze infiziert sind. Auch Zellen, die antigene Teile dieser Fremdorganismen oder Tumorzellen enthalten, können durch das erfindungsgemäße Verfahren lysiert werden, wobei die Lysis dadurch vermittelt wird, daß die Sensitivität dieser Zielzellen für den Angriff durch NK-Zellen erhöht wird.

Das erfindungsgemäße Verfahren, das eine Steigerung der Sensitivität der Zielzellen für die Lysis durch Natürliche Killerzellen erreicht, ist dadurch gekennzeichnet, daß eine zumindest die Zielzellen und die natürlichen Killerzellen enthaltende physiologische Zellsuspension kombinatorisch in einer bestimmten, genau einzuhaltenden Reihenfolge zunächst einer Hitzebehandlung unterzogen wird, worauf, nach einer Erholungsphase, eine Behandlung mit einer subletalen Konzentration einer Verbindung erfolgt, die den membrangebundenen Hsp70-Anteil der Zielzellen erhöht. Als eine den membrangebundenen Hsp70-Anteil der Zielzellen erhöhenden Verbindung wird erfindungsgemäß ein Alkyl-Lysophospholipid eingesetzt. Hierauf schließt sich eine weitere Erholungsphase an. Bei Anwendung des erfindungsgemäßen Verfahrens wird erreicht, daß die Zielzellen für eine Lysis durch NK-Zellen deutlich sensitiver werden.

Die NK-Zellen können durch geeignete Verfahren aus den zu behandelnden Patienten oder aus einem gesunden Spender isoliert werden. Bevorzugt liegen jedoch die NK-Zellen zusammen mit anderen peripheren, mononukleären Blutzellen vor, beispielsweise in Form von Buffy-Coat-Zellen.

Buffy-Coat-Zellen werden über die Vene aus dem Patienten entnommen und z.B. mit Heparin versetzt, um eine Verklumpung der Zellen zu verhindern. Das mit Heparin versetzte Vollblut wird in einem sterilem Gefäß (zumeist Plastiksäckchen) gesammelt und anschließend zentrifugiert, so daß es zu einer Anreicherung von Blutzellen ( = PBMC, periphere, mononukleäre Zellen, z.B. Lymphozyten, Erythrozyten, Granulozyten usw.) kommt. Ein großer Teil des Serums (2/3) wird steril entnommen und für den Patienten aufbewahrt, der sich beispielsweise einer Operation unterzieht und gegebenenfalls sein eigenes Serum benötigt. Das Lymphozytenkonzentrat bleibt im Gefäß (Plastikbeutel). Im Falle von gesunden Probanden besteht ein Buffy-Coat aus weißen und roten Blutzellen (Lymphozyten, Erythrozyten usw.). Im Falle eines Tumorpatienten besteht der Buffy-Coat nicht nur aus Blutzellen, sondern enthält auch Tumorzellen (bei Leukämien, z.B. leukämische Zellen = Blasten; bei soliden Tumoren, z.B. Metastasen als Einzelzellen).

Das Vollblut oder die Buffy-Coat-Zellen, die periphere, mononukleäre Blutzellen enthalten, werden in Form einer physiologischen Zellsuspension, bevorzugt versetzt mit Heparin, eingesetzt. Das Heparin verhindert eine Aggregation der Zellen.

Völlig unerwartet konnte gezeigt werden, daß die Zielzellen, hier gezeigt am Beispiel von K562-Leukämiezellen, die eine starke Resistenz gegenüber einer Behandlung mit einer Verbindung, die den membrangebundenen Hsp70-Anteil in der Zielzelle erhöht, beispielsweise einem Alkyl-Lysophospholipid, aufweist, durch die Kombination von Hitze- und ALP-Behandlung in der angegebenen Reihenfolge signifikant besser lysierbar sind.

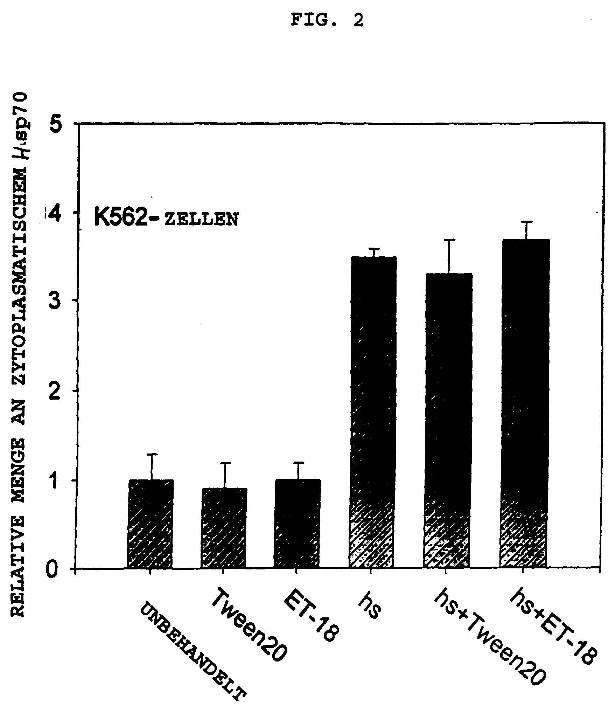

Insbesondere konnte gezeigt werden, daß bei einer gleichzeitigen Durchführung der Hitzebehandlung und der Alkyl-Lysophospholipid-Behandlung eine synergistische Stimulation der Lysis durch Natürliche Killerzellen nicht erfolgte. Der Erfolg stellte sich auch nicht ein, wenn zuerst eine Behandlung mit z. B. einem Alkyl-Lysophospholipid erfolgte und sich dann eine Hyperthermie-Behandlung anschloß (Fig. 2).

Erst die Kombination beider Verfahren in der erfindungsgemäß beanspruchten Reihenfolge, nämlich zunächst Hitzebehandlung und dann Behandlung mit einer subletalen Konzentration z.B. eines Alkyl-Lysophospoholipids, wobei nach der Hitzebehandlung und nach der Alkyl-Lysophospholidbehandlung je eine ausreichende Erholungsphase von mindestens einer Stunde für die Zellen einzulegen ist, führt zum für einen Patienten hoch-gewünschten, jedoch aufgrund des bekannten Standes der Technik unerwarteten Erfolg. Bei der Ausarbeitung der erfindungsgemäß durchgeführten Experimente zeigte es sich auch, daß die relative Zunahme membrangebundenen Hsp70-Proteins bei einer Kombination der Alkyl-Lysophospholipid-Behandlung und der Hitzebehandlung nicht nur additiv war, sondern überadditiv, also synergistisch. Eine derartige synergistische Zunahme an membrangebundenem Hsp70 durch das erfindungsgemäße Verfahren konnte nicht vorausgesehen werden. Es wird darauf hingewiesen, daß das Hsp70-Protein dem Hsp72-Protein entspricht, und das am stärksten hitzeinduzierbare Protein der Gruppe der HSP70-Familie darstellt.

Bei der erfindungsgemäßen Behandlung, also einer Kombination von Hitzebehandlung und z.B. Alkyl-Lysophospholipidbehandlung, kommt es ausschließlich bei den Zielzellen zu einer Membranexpression von Hsp70, wohingegen die normalen Blutzellen, auch die von Patienten, keine oder zumindest keine erhöhte Hsp70-Membranexpression aufweisen. Die Expression von Hsp70 auf der Zellmembran der Zielzellen, z.B. Tumorzellen stellt ein Erkennungssignal für die Lysis durch NK-Zellen dar.

Die Hitzebehandlung der Zielzellen, die in einer Kulturlösung zusammen mit Natürlichen Killerzellen (NK-Zellen) vorliegen, erfolgt durch Erhöhung der Temperatur auf 38°C bis 43°C, beispielsweise auf 40°C bis 42°C. Höhere Temperaturen werden bevorzugt. Jedoch darf die Temperatur selbstverständlich nicht so hoch liegen, daß die Zellen letal geschädigt werden, da zu viele tote Zellen nicht in den Patienten reinfundiert werden dürfen. Die Hitzebehandlung wird in einem solchen Zeitraum durchgeführt, der ausreichend ist, um die Bildung von Hitzeschockproteinen, bevorzugt Hsp70, in der Zelle zu induzieren. Bevorzugt erfolgt die Hitzebehandlung für mindestens eine Stunde, wobei sich eine Zeit von bis zu drei Stunden als günstig erwiesen hat. In Abhängigkeit von den zu behandelnden Zielzellen sind jedoch auch andere Zeiten und andere Temperaturen einsetzbar. Diese können vom Fachmann durch Versuche ohne erfinderisches Zutun ermittelt werden.

Die Erholungsphasen sind zwingend einzuhalten. Die Erholungsphase nach der Hitzebehandlung soll mindestens eine Stunde und die Erholungsphase nach der Behandlung mit z.B. einem Alkyl-Lysophospholipid mindestens eine Stunde betragen, und sie können sich auf beispielsweise auf bis zu 16 Stunden ausdehnen. Es hat sich herausgestellt, daß bevorzugte Erholungsphasenzeiten von 8 bis 12 Stunden günstig sind. Jedoch können auch, in Abhängigkeit von den zu behandelnden Zellen, andere Zeiten eingesetzt werden. Die Temperatur während der Erholungszeit entspricht bevorzugt der physiologischen Temperatur der Zellen, bevorzugt etwa 37°C.

Die Behandlung der Zielzellen mit z.B. dem Alkyl-Lysophospholipid erfolgt mit einer Konzentration, die im subletalen Bereich liegt, so daß die Zellen nicht letal geschädigt werden. Die subletale Konzentration des Alkyl-Lysophospholipids liegt bei den erfindungsgemäß beispielhaft eingesetzten K562-Zellen in einem Bereich von 10-30 µg/ml. Ein günstiger Bereich liegt bei 15-25 µg/ml, weiterhin bei 15-20 µg/ml oder bei 10-25 µg/ml. In Abhängigkeit von den zu behandelnden Zielzellen sind auch andere Konzentrationen einsetzbar, die durch Versuche ohne erfinderisches Zutun ermittelbar sind.

Für das erfindungsgemäße Behandlungsverfahren werden bevorzugt Alkyl-Lysophospholipide eingesetzt. Alkyl-Lysophospholipide sind an sich bekannt und werden beispielsweise in (10) beschrieben. Beispiele hierfür sind ES-18-OCH3 (Lysophosphatidylcholin), BN52205, BN52207, BN52208, BN52211. Es ist jedoch auch möglich andere Verbindungen als die genannten Alkyl-Lysophospholipide einzusetzen, vorausgesetzt sie sind in der Lage, den membrangebundenen Hsp70-Anteil der Zielzellen zu erhöhen.

Durch das erfindungsgemäße Verfahren können Tumorzellen abgetötet werden. Bevorzugt werden dabei Leukämiezellen und Tumorzellen aus Metastasen solider Tumoren lysiert. Beispiele für Leukämiezellen sind K562-Zellen oder Lymphomzellen. Beispiele für Tumorzellen von Metastasen solider Tumoren sind Karzinomzellen, Sarkomzellen und Melanomzellen. Beispiele hierfür sind Ovarialkarzinomzellen, Kolonkarzinomzellen, Adenokarzinomzellen, Mammakarzinomzellen und epidermoide Karzinomzellen. Weiterhin können durch das erfindungsgemäße Verfahren auch menschliche oder tierische Zellen abgetötet bzw. lysiert werden, die durch Viren, Bakterien und/oder Pilze infiziert sind. Diese Zellen werden allgemein als Zielzellen bezeichnet. Weiterhin können auch solche Zellen durch die durch NK-Zellen vermittelte Immunantwort angegriffen werden, die antigene Teile dieser Viren, Bakterien und/oder Pilze oder Tumorzellen enthalten.

Beispiele für durch Viren infizierte Zellen sind HIV-infizierte Zellen, Beispiele für die durch Bakterien infizierten Zellen sind durch Mycobakterien infizierte Zellen.

Nachfolgend wird die Erfindung anhand von Ausführungsbeispielen und der beiliegenden Zeichnungen näher beschrieben. Die Ausführungsbeispiele dienen lediglich zur Veranschaulichung der Erfindung, und die Erfindung ist nicht auf diese Beispiele beschränkt. Abwandlungen der Erfindung im Rahmen der vorliegenden Beschreibung und der Patentansprüche sind selbstverständlich möglich und werden durch die erfindungsgemäßen Ansprüche mitumfaßt.

Die beiliegenden Abbildungen und Tabellen zeigen:
- Tabelle 1:: Die Überlebensfähigkeit von K562-Leukämiezellen im Vergleich zu PBL-Zellen und CD34-positiven Progenitorzellen aus gesunden Spendern nach einer kombinatorischen Behandlung mit einer nichtletalen Hitzedosis (bei 41,8°C, 2 Stunden) und ET-18-OCH3 oder Tween20 bei verschiedenen Konzentrationen.
Die Überlebensfähigkeit ist als Prozentsatz überlebender Zellen angegeben und wird durch Trypanblau-Farbstoff-Ausschlußfärbungen bestimmt. CD34-positive Progenitorzellen wurden nicht mit Tween20 behandelt, da die Menge der aus einem Spender abgetrennten Zellen für alle Tests nicht ausreichte. Die Werte stellen Mittelwerte dreier unabhängiger Experimente dar; n.t. bedeutet "not tested", d.h. es wurden keine Versuche durchgeführt.
- Tabelle 2:: Die Membranexpression von Hsp70 auf PBL- und CD34-positiven Progenitorzellen (Reinheit > 90%), entweder ohne Behandlung oder mit einer nachfolgenden Behandlung mit ET-18-OCH3, 25 µg, Hitze (41,8°C, 2 Stunden) oder Hitze plus ET-18-OCH3. Die Ergebnisse werden ausgedrückt als Differenz im Prozentsatz an Hsp70-positiv gefärbten Zellen minus dem Prozentsatz an Zellen, die mit einem dem Isotyp entsprechenden Kontrollantikörper gefärbt sind. Nur lebensfähige, Propidiumiodid-negative Zellen wurden auf einem FACScan-Gerät analysiert. Die angegebenen Werte stellen Mittelwerte dreier unabhängiger Versuche ± Standardabweichung (SD) dar.
- Tabelle 3:: zeigt die Berechnung der spezifischen Unterschiede bei der Lysis von K562-Zellen, entweder unbehandelt (untr), hitzebehandelt (hs), mit ET-18-OCH3-behandelt oder behandelt mit Hitze und ET-18-OCH3. Die Werte zeigen Mittelwerte dreier Experimente ± Standardabweichung (SD). * bedeutet Werte, die sich signifikant (p < 0,05) von den Kontrollwerten, berechnet durch den t-Test von Student, unterscheiden.
- Tabelle 4:: zeigt die Berechnung der spezifischen Unterschiede in der Lysis von PBL- und CD34-angereicherten Zellen (CD34-positive Zellen lagen im Bereich von 25%-45%), entweder ohne Behandlung (untr) oder mit einer Behandlung durch Hitze und ET-18-OCH3. Die Werte zeigen die Mittelwerte zweier Experimente ± Standardabweichung (SD).

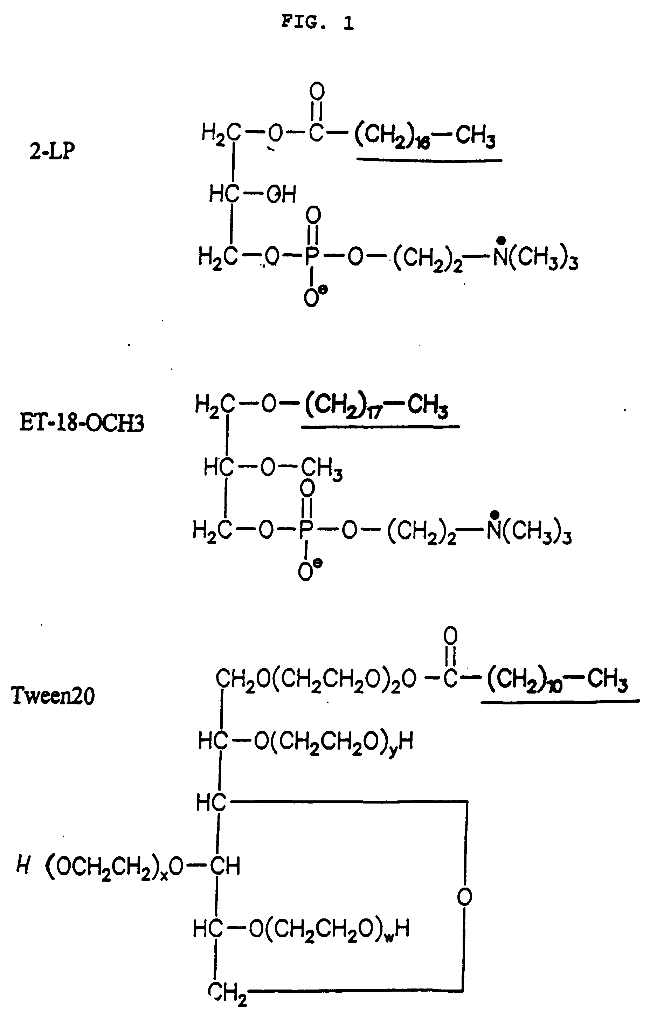

- FIGUR 1:: Die Figur 1 zeigt die chemische Struktur von 2-Lysophosphatidylcholin (2-LP), 1-Octadecyl-2-methyl-racglycero-3-phosphocholin (ET-18-OCH3) und Polyoxyethylensorbat 20 (Tween20). Möglicherweise mit der Membran interagierende Bereiche sind fettgedruckt und unterstrichen.
- FIGUR 2:: Weder durch eine Hitzebehandlung noch durch eine Behandlung mit Tween20 kann der zytoplasmatische Anteil von Hsp70 in leukämischen K562-Zellen erhöht werden. Ein Vergleich der relativen Menge von Hsp70 im Zytoplasma von K562-Zellen zeigt:
unbehandelt (Spur 1), behandelt mit Tween20 (5 ppm, 2 Stunden; Spur 2), ET-18-OCH3-behandelt (25 µg/ml, 2 Stunden; Spur 3), hitzebehandelt (41,8°C, 2 Stunden; Spur 4), behandelt mit Hitze und Tween20 (Spur 5) oder behandelt mit Hitze und ET-18-OCH3 (Spur 6), oder mit ET-18 und Hitze (Spur 7). Die Zellysate wurden auf einem 10% SDS-PAGE unter reduzierenden Bedingungen aufgetrennt und auf eine PVDF-Membran übertragen. Hsp70 wurde mit einem Hsp70-spezifischen Antikörper nachgewiesen; die Immunoblots wurden durch Laser-Densitometrie quantifiziert. Die Ergebnisse stellen Mittelwerte dreier unabhängiger Experimente dar; die vertikalen Linien stellen die Standardabweichung dar.

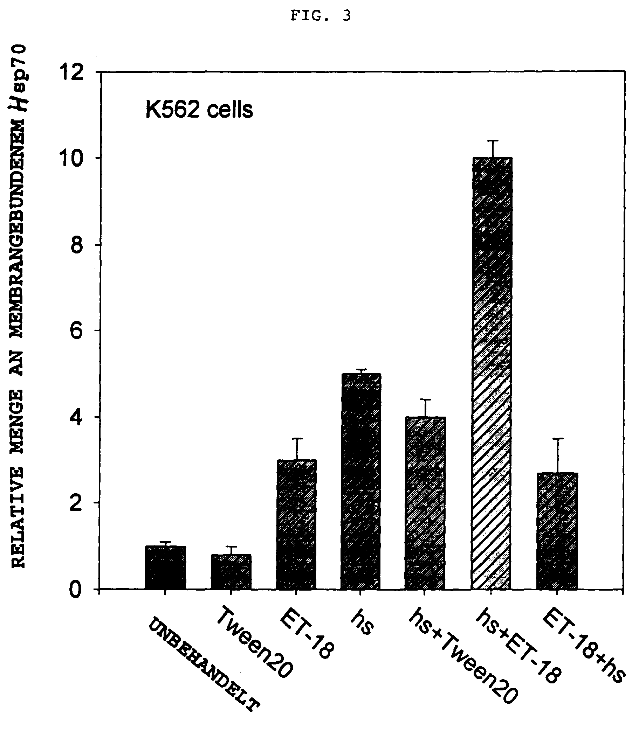

- FIGUR 3:: Synergistischer Einfluß von Hitze und ET-18-OCH3 auf die Membranexpression von Hsp70. Vergleich der relativen Menge von membrangebundenem Hsp70 in K562-Zellen:
unbehandelt (Spur 1), behandelt mit Tween20 (5 ppm, 2 Stunden; Spur 2), behandelt mit ET-18-OCH3 (25 µg/ml, 2 Stunden; Spur 3), hitzebehandelt (41,8°C, 2 Stunden; Spur 4), behandelt mit Hitze und Tween20 (Spur 5) oder behandelt mit Hitze und ET-18-OCH3 (Spur 6) oder mit ET-18-OCH3 und Hitze (Spur 7). Gleiche Proteinmengen (10 µg) aus K562-Membranlysaten wurden auf einem 10% SDS-PAGE unter reduzierenden Bedingungen aufgetrennt und auf PVDF-Membran übertragen. Die Bande des 72 kD-Hitzeschockproteins wurde mit einem Hsp70-spezifischen, monoklonalen Antikörper nachgewiesen und durch das ECL-System detektiert. Die Immunoblots wurden mit Laser-Densitometrie quantifiziert. Die Ergebnisse zeigen die Mittelwerte zweier unabhängiger Experimente; die vertikalen Linien stellen die Standardabweichung dar.

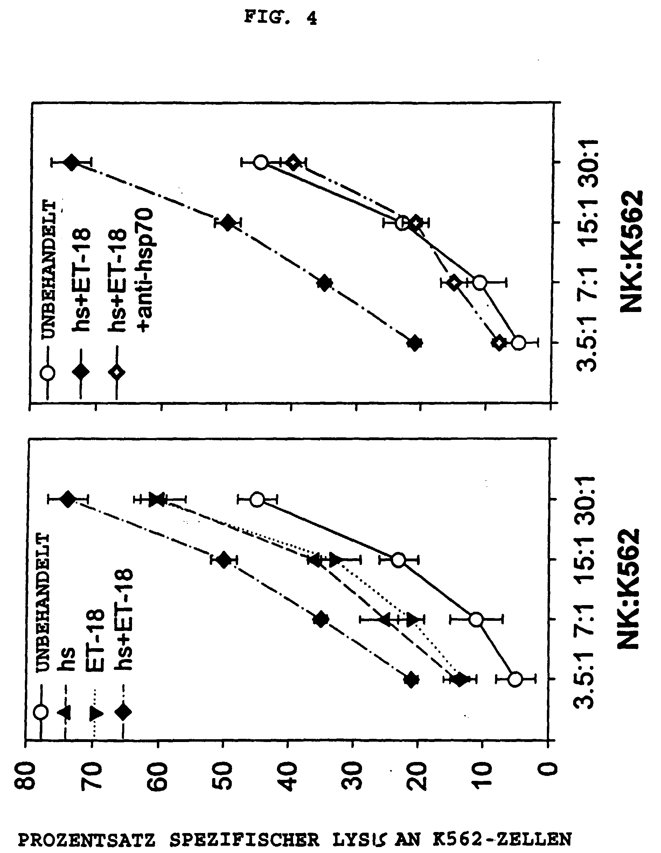

- FIGUR 4:: Synergistische Zunahme der Sensitivität für die Lysis nach Behandlung mit Hitze und ET-18-OCH3. Ein Vergleich der zytotoxischen Aktivität einer NK-angereicherten Effektorzellpopulation gegenüber K562-Leukamiezellen nach Hitzebehandlung (hs; 41,8°C, 2 Stunden), Tween20-Behandlung (5 ppm), Behandlung mit Hitze plus ET-18-OCH3 (hs + ET-18; 25 µg/ml) oder Behandlung durch Hitze plus Tween20 (hs + Tween20). Die Verhältnisse von E:T (NK:K562) reichten von 3,5:1 bis 30:1. Jeder Wert stellt den Mittelwert dreier unabhängiger Experimente dar. Eine statistische Analyse der Unterschiede in der Lysis von K562-Zellen ist in Tabelle 2 angegeben.

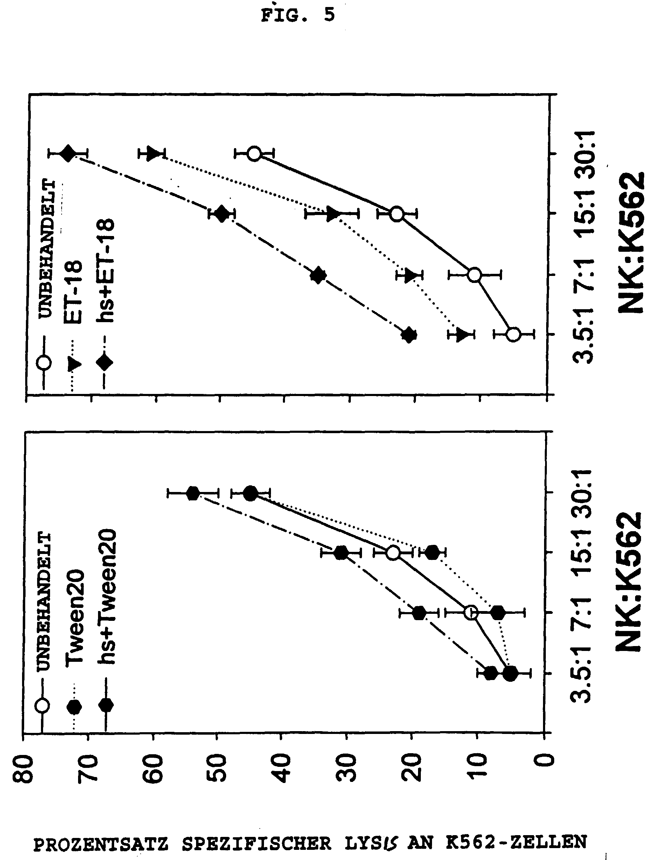

- FIGUR 5:: Die synergistische Zunahme der Sensitivität für die Lysis durch die erfindungsgemäße Kombinationsbehandlung mit Hitze und ET-18-OCH3 kann durch Anwendung eines Hsp70-spezifischen Antikörpers blockiert werden. Ein Vergleich zeigt die zytotoxische Aktivität einer mit NK-Zellen angereicherten Effektorzellpopulation gegen unbehandelte, mit Hitze (hs; 41,8°C, 2 Stunden) oder mit Hitze und ET-18-OCH3 (hs + ET-18, 25 µg/ml) behandelte K562-Leukämiezellen. Die E:T-Verhältnisse (NK:K562) reichten von 3,5:1 bis 30:1. Jeder Wert stellt den Mittelwert dreier unabhängiger Experimente dar. Eine statistische Analyse der Unterschiede der Lysis von K562-Zellen ist in Tabelle 2 angegeben.

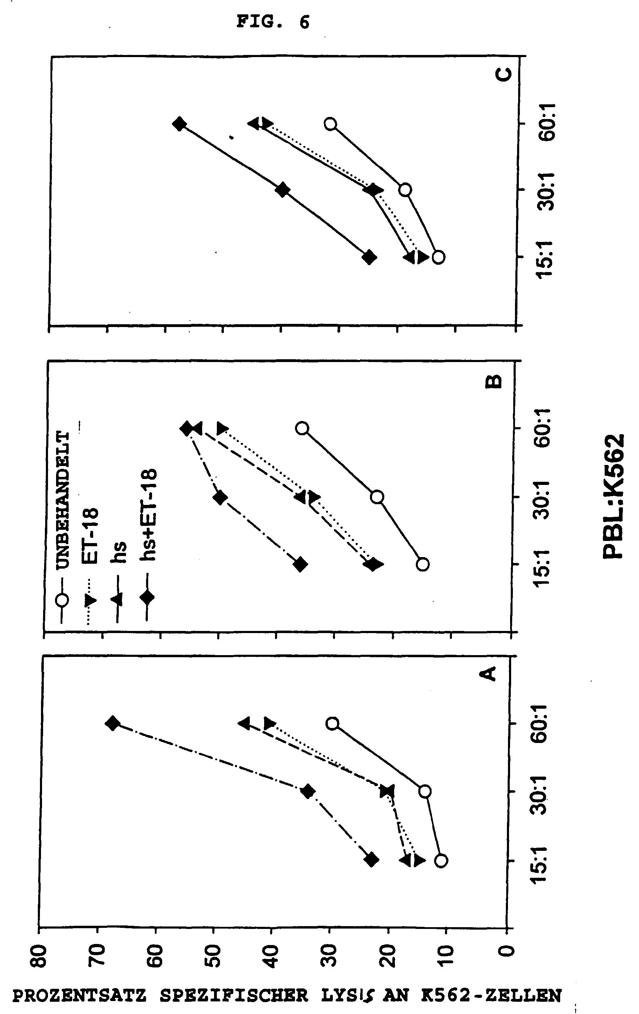

- FIGUR 6:: Erhöhte Lysis von mit Hitze und/oder ET-18 behandelten K562-Leukämiezellen wird durch unstimulierte, periphere Blutlymphozyten (ruhende NK-Zellen) vermittelt. Ein Vergleich der zytotoxischen Aktivitäten nicht stimulierter Effektorzellen, die aus drei HLA-verschiedenen, menschlichen Spendern stammten (A,B und C), gegen unbehandelte, hitzebehandelte (hs; 41,8°C, 2 Stunden) oder hitze- und ET-18-0CH3 (hs + ET-18; 25 µg/ml) behandelte K562-Leukämiezellen ist dargestellt. Die E:T-Verhältnisse (PBL:K562) reichten von 15:1 bis 60:1.

Erfindungsgemäß wurde überraschend festgestellt, daß durch die kombinierte Behandlung von Zielzellen, z.B. Tumorzellen, mit einer erhöhten Temperatur und einer subletalen Konzentration z.B. eines Alkyl-Lysophospholipids in der angegebenen Reihenfolge auch solche Zielzellen, z.B. Tumorzellen, beispielsweise Leukämiezellen, für eine Zellyse durch Natürliche Killerzellen (NK-Zellen) empfänglich werden, die ohne diese kombinierte Behandlung durch NK-Zellen nicht angegriffen werden. Während gemäß dem Stand der Technik Alkyl-Lysophospholipide in solchen Konzentrationen eingesetzt werden, die eine zellschädigende, d.h. direkte, zytotoxische Wirkung zeigen, um so die Zielzellen direkt abzutöten, wird erfindungsgemäß ein wesentlich sanfteres Verfahren eingesetzt, das keine direkte zytotoxische Wirkung auf die Zielzellen ausübt, sondern indirekt über NK-Zellen die Zielzellen durch Lysis tötet.

Aufgrund der erfindungsgemäß durchgeführten Versuche ist davon auszugehen, daß durch die kombinierte Behandlung aus einem subletalen Hitzeschock und einer nicht-zytotoxischen Konzentration eines Alkyl-Lysophospholipids der Anteil membrangebundener Hitzeschockproteine, insbesondere des membrangebundenen Hitzeschockproteins Hsp70, auf den Zielzellen, hier gezeigt anhand von Leukämiezellen, nicht nur additiv, sondern synergistisch zunimmt. Diese Zunahme an membrangebundenem Hsp70 ist aller Wahrscheinlichkeit nach dafür verantwortlich, daß bei der erfindungsgemäßen Kombinationsbehandlung der Zielzellen durch Hitze und Alkyl-Lysophospholipid in der angegebenen Reihenfolge eine synergistische Zunahme der Sensitivität der Zielzellen für die Lysis durch NK-Zellen erfolgt (Antikörperblockierungsexperimente belegen diesen Befund), (vgl. Figuren 4 und 5).

Die NK-Zellen, die die Lysis der Zielzellen vermitteln, sind insbesondere dadurch gekennzeichnet, daß sie CD16 exprimieren und/oder durch Interleukin-2 stimulierbar sind und/oder kein CD3 exprimieren und/oder keine α/β- oder γ/δ- T-Zell-Rezeptoren besitzen und/oder nicht vom MHC-Typ des Patienten abhängig sind.

Die NK-Zellen, die im erfindungsgemäßen Verfahren ihre Wirksamkeit entfalten, sind weiterhin bevorzugt durch die nachfolgenden Charakteristika gekennzeichnet:

### NK-Zell-Charakteristika:

Transient plastik-adhärent nach Zugabe von IL-2 (Chiron, 100 I.U.):
- Die Adhärenz erfolgt 12-18 Stunden nach Zugabe des IL-2 auf frisch isolierte PBL (monozytendepletierte, periphere Blutlymphozyten);
- die NK-Zellen weisen eine CD16dim Expression auf (mean Fluorszenz schwach);
- die NK-Zellen exprimieren CD56 und CD57 als NK-Marker;
- Die NK-Zellen exprimieren CD94 (C-Typ Lectin Killer-Zell-Rezeptor);
- die NK-Zellen sezernieren nach Aktivierung IFNgamma;
- nicht vom MHC-Typ des Patienten abhängig.

Es können auch andere NK-Zellpopulationen eingesetzt werden, vorausgesetzt, sie lysieren nach der erfindungsgemäßen Behandlung die Tumorzellen.

Das erfindungsgemäße Verfahren eignet sich bevorzugt zur Behandlung von Leukämiezellen. Es können jedoch auch andere Tumorerkrankungen behandelt werden, bevorzugt Erkrankungen mit soliden Tumoren, beispielsweise Ovarialkarzinomen, Kolonkarzinomen, Adenokarzinomen, epidermoiden Karzinomen und Mammakarzinomen. Es können aber auch andere Zielzellen als Angriffspunkte dienen. Beispiele hierfür wurden vorstehend genannt.

Durch das erfindungsgemäße Verfahren eröffnet sich die Möglichkeit, nicht nur ex vivo im Rahmen einer Purging-Behandlung Tumorzellen, z.B. Leukämiezellen, direkt abzutöten, sondern aufgrund der Vermeidung zytotoxischer Konzentrationen des Etherlipids das Verfahren in Kombination mit einer Hyperthermiebehandlung auch in vivo einzusetzen. Das erfindungsgemäße Verfahren hat den weiteren, unschätzbaren und überraschenden Vorteil, daß auch Zielzellen, z.B. Tumorzellen, die den bekannten Verfahren einer Hyperthermiebehandlung und einer Etherlipidbehandlung widerstanden, nunmehr durch die indirekte Wirkung über NK-Zellen abgetötet werden können.

Ein Beispiel für die Behandlung mit dem erfindungsgemäßen Verfahren ist wie folgt:
Buffy-coat-Zellen (Lymphoztenkonzentrate), bestehend aus peripheren, mononukleären Blutzellen oder Knochenmarkszellen und Tumorzellen aus Tumorpatienten, beispielsweise Leukämiepatienten, werden in einem Behälter, beispielsweise einem Kunststoffbehälter, der steril verschlossen ist, einer Hyperthermiebehandlung in einem temperaturkontrollierten Wasserbad unterzogen. Nach einer Erholungsphase, die in einem Brutschrank oder einem Wasserbad bei der Erholungstemperatur (bevorzugt bei der physiologischen Temperatur der Zellen, also bei etwa 37°C) durchführbar ist, wird das Alkyl-Lysophospholipid in einer subletalen Konzentration in den Behälter zugegeben. Im Behälter befinden sich sowohl die Tumorzellen als auch die NK-Zellen, die über die vorliegende Behandlung stimuliert werden. Nach Abschluß des erfindungsgemäßen Verfahrens wird die NK-Zellen und die lysierten Tumorzellen enthaltende Kulturlösung in den Patienten reinfundiert.

Erfindungsgemäß liegen, wie bereits weiter oben ausführlich dargestellt, die NK-Zellen zusammen mit anderen peripheren, mononukleären Blutzellen, beispielsweise zusammen mit Erythrozyten und Granolozyten und T-Zellen, vor. Bevorzugt werden somit die NK-Zellen nicht alleine verwendet, sondern durch Isolierung von Buffy-Coat-Zellen und Anreicherung der peripheren, mononukleären Blutzellen gewonnen. Bei Tumorpatienten enthalten diese Anreicherungen weiterhin Tumorzellen, die durch das erfindungsgemäße Verfahren behandelt werden.

Die Behandlungsstrategie zielt insbesondere auf die Elimination von Einzelzell-Metastasen ab, die durch das erfindungsgemäße Verfahren immunologisch eliminiert werden können. Eine verstärkte Aktivierung Hsp70-spezifischer NK-Zellen kann durch eine Zugabe von Interleukin-2 in einer niedrigen Dosis, beispielsweise 100 I.U., erreicht werden. Das Interleukin-2 kann beispielsweise zusammen mit dem Alkyl-Lysophospholipid in den sterilen Behälter, beispielsweise einen Kunststoffbehälter, eingeführt werden.

Das erfindungsgemäße Verfahren wurde anhand eines speziellen Etherlipids, nämlich ET-18-OCH3, einem synthetischen Derivat des natürlich vorkommenden 2-Lysophosphatidylcholins, in Verbindung mit der Leukämiezellinie K562 entwickelt. Es ist aber auch auf andere Leukämiezellen, allgemein auch auf andere Tumorzellen, und mit anderen Alkyl-Lysophospholipiden, durchführbar.

Um die nicht-toxischen Konzentrationen von ET-18-OCH3 in Kombination mit einer nicht-letalen Temperatur für periphere Blutlymphozytenzellen (PBMC-Zellen), K562-Zellen und CD34-positive Stammzellen zu untersuchen, wurden Trypanblau- oder Propidiumiodid-Ausschlußuntersuchungen durchgeführt. Die Tabelle 1 zeigt, daß eine Konzentration von 25 µg/ml mit ET-18-OCH3 in einem 10% FKS-enthaltenden Medium und eine Inkubationszeit von 2 Stunden für K562-Zellen nicht toxisch waren. Sowohl PBL-Zellen als auch CD34-positive Progenitorzellen aus gesunden Spendern zeigten eine ähnliche Resistenz gegen eine Behandlung mit ET-18-OCH3. Es wurde weiterhin gezeigt, daß diese nicht-toxische Konzentration an ET-18-OCH3 bei K562-Zellen keine Apoptose induziert. Als Kontrolle wurde das membran-interaktive Detergens Tween20 verwendet. Es konnte gezeigt werden, daß bei einer Inkubationszeit von 2 Stunden und einer Konzentration von 5 ppm auf K562-Zellen, PBL-Zellen und CD34-Zellen keine zytotoxische Wirkung erfolgte (vgl. Tabelle 1). Für alle weiteren Untersuchungen wurde diese nicht-letale Konzentration ausgewählt.

Um festzustellen, ob eine Kombination aus einer nicht-letalen Hitzebehandlung, nicht-toxischen Konzentrationen an ET-18-OCH3 (25 µg/ml für 2 Stunden) und Tween20 (5 ppm für 2 Stunden) entweder allein oder in Kombination mit einer Hitzebehandlung (41,8°C für 2 Stunden) die Synthese von Hitzeschockproteinen (Hsp) induzierte, wurde der Hsp70-Gehalt im Zytoplasma bestimmt. Es wurde gefunden, daß weder Tween20 noch ET-18-OCH3 für sich allein den Gehalt von Hsp70 im Zytoplasma erhöhten. Wenn die K562-Zellen ausschließlich einer Hitzebehandlung ausgesetzt wurden, ergab sich eine etwa 2-fache Zunahme des zellulären Hsp70-Spiegels in den K562-Zellen. Diese Zunahme wurde auch dann nicht beeinflußt, wenn die Zellen einer kombinierten Behandlung von Hitze und Tween20 oder Hitze und ET-18-OCH3 ausgesetzt wurden (vgl. Figur 2). Ähnliche Ergebnisse konnten sowohl mit PBL-Zellen aus gesunden Spendern oder CD34-positiven Vorläuferzellen erzielt werden. Aufgrund der geringeren Anfangsgehalte an Hsp70 in PBL-Zellen und CD34-Vorläuferzellen betrug die Zunahme von Hsp70 nach der Hitzebehandlung im Zytoplasma mehr als das 5-fache (Daten nicht gezeigt).

Im Gegensatz zur Induktion von Hsp70 im Zytoplasma erhöhte sich der membrangebundene Hsp70-Anteil nach Behandlung mit Hitze (41,8°C für 2 Stunden), einer Erholungsphase bei 37°C, für 8 Stunden und/oder ET-18-OCH3 (25 µg/ml, 2 Stunden) und einer Erholungsphase von 2 Stunden bei 37°C signifikant. Eine Behandlung mit ET-18-OCH3 allein erhöht den membrangebundenen Hsp70-Anteil um das etwa 3-fache, die Hitzebehandlung um das 5-fache und bei einer gleichzeitigen Behandlung der Zellen mit Hitze plus ET-18-OCH3 ergibt sich eine mehr als 10-fache Zunahme des Anteils membrangebundenen Hsp70. Eine Behandlung der K562-Zellen mit ET-18-OCH3 und anschließender Hitzebehandlung führt im Vergleich zu alleiniger Hitzebehandlung zu keiner weiteren Steigerung der Hsp70-Expression an der Membran (Figur 3), d.h. die Reihenfolge der Behandlung ist ausschlaggebend für den Erfolg. Die Behandlung der Zellen mit dem membran-interaktiven Tween20 (5 ppm, 2 Stunden) zeigte alleine keinen Einfluß auf den Anteil an membrangebundenem Hsp70. In Kombination mit einer Hitzebehandlung war der Effekt nicht höher als mit Hitze alleine.

Durchflußzytometrische Untersuchungen zeigten, daß PBL-Zellen oder CD34-positive Vorläuferzellen im Gegensatz zu K562-Zellen weder unter physiologischen Bedingungen noch nach einer Behandlung mit Hitze und/oder ET-18-OCH3 eine Hsp70-Plasmamembran-Expression zeigten (vgl. Tabelle 2). Dies beweist, daß es sich bei der Membranexpression von Hsp70 um ein tumorspezifisches Phänomen handelt.

Um die Frage zu beantworten, ob die mehr als 10-fache Zunahme des Anteils an membrangebundenen Hsp70 in K562-Zellen durch eine kombinierte Behandlung mit Hitze und ET-18-OCH3, eine gesteigerte Sensitivität der Tumorzellen für eine Lysisbehandlung induziert, wurden Zytotoxizitätstests durchgeführt. Als Effektorzellen wurden ruhende NK-Zellen ( = unstimulierte PBL) oder mit IL-2 stimulierte, NK-angereicherte Zellpopulation verwendet. Als Vergleichstest wurde ein Chromfreisetzungstest verwendet, der sowohl bei unbehandelten K562-Zellen als auch bei K562-Zellen durchgeführt wurde, die einer kombinierten Behandlung aus Hitzeschock plus ET-18-OCH3 ausgesetzt waren. Die kombinierte Behandlung mit Hitzeschock und ET-18-OCH3 zeigte eine signifikante (p < 0,05) Zunahme in der Sensitivität der Tumorzellen für eine Lysis im Vergleich zu den Kontrollen (etwa 2-fache Zunahme). Die Lysis von K562-Zellen, die nur mit ET-18-OCH3 behandelt worden waren, erhöhte sich um das etwa 1,3-fache (Tabelle 3); bei Tumorzellen, die ausschließlich mit Tween20 behandelt wurden, konnte keine Zunahme im Vergleich zu den Kontrollen festgestellt werden. Weiterhin ergab auch eine kombinierte Behandlung von K562-Zellen mit Hitze plus Tween20 keine erhöhten Lysisanteile im Vergleich zur Lysis mit hitzeschockbehandelten K562-Zellen (je 1,3-fach; Figur 3 und 4).

Erfindungsgemäß konnte somit eine synergistische Wirkung bei einer kombinierten, nacheinanderfolgenden Behandlung von K562-Zellen mit Hitze und ET-18-OCH3 festgestellt werden. Hier ist zu bemerken, daß die Reihenfolge der Behandlung: zuerst Hitzeschock, dann Erholungsphase bei etwa 37°C und anschließend Behandlung mit dem Alkyl-Lysophospholipid, verbunden mit einer weiteren Erholungsphase bei etwa 37°C, von entscheidender Bedeutung für den Erfolg ist. In umgekehrter Reihenfolge und bei Zugabe des Alkyl-Lysophospholipids während der Hyperthermiebehandlung ergab sich kein synergistischer Effekt. Es zeigte sich, daß der Anteil an in der Plasmamembran lokalisiertem Hsp70 deutlich zunahm (vgl. Figur 3) und sich diese Zunahme in einer erhöhten Sensitivität der so behandelten Tumorzellen gegenüber einer Lysis durch NK-Zellen widerspiegelte (Figur 5). Antikörper-Blockierinngsuntersuchungen unter Verwendung eines Hsp70-spezifischen Antikörpers zeigen, daß die erhöhte Lysis-Sensitivität mit der Menge an membrangebundenem Hsp70 korreliert (vgl. Figur 5). Bei unbehandelten oder mit Tween20 behandelten K562-Zellen konnte keine erhöhte lytische Aktivität festgestellt werden (Daten nicht gezeigt).

CD34-positive Vorläuferzellen oder PBL-Zellen, von denen gezeigt werden konnte, daß sie Hsp70 auf ihrer Plasmamembran nicht exprimieren, konnten durch NK-Zellen weder unter physiologischen Bedingungen noch unter den erfindungsgemäß durchgeführten Behandlungen unter Hitzestreß oder Hitze und ET-18-OCH3-Streß lysiert werden (vgl. Tabelle 4). Dies zeigt, daß es sich bei dem durch das erfindungsgemäße Verfahren vermittelten Effekt um eine tumorspezifische Wirkung handelt.

Neben der mit IL-2 stimulierten, NK-angereicherten Zellpopulation wurde die Zytotoxizitätsaktivität von unstimulierten, ruhenden NK-Zellen (PBL) von drei HLA-verschiedenen Spendern untersucht. Die Lysis-Muster aller drei HLA-verschiedener Effektorzellpopulationen (A, B und C) waren vergleichbar (Figur 6) und ergaben ähnliche Ergebnisse wie diejenigen, die bei den mit IL-2 stimulierten NK-Zellen erhalten wurden. Wiederum führte eine Hitzebehandlung (2 Stunden, 41,8°C) als auch eine Behandlung mit ET-18-OCH3 (25 µg/ml, 2 Stunden) mit einer nachfolgenden Erholungsdauer von 2 Stunden bei 37°C von K562-Zellen zu einer signifikanten Zunahme (etwa das 1,4-fache) in der Sensitivität auf Lysis, wobei die Lysis nach der Behandlung der K562-Zellen mit einer Kombination aus Hitzeschock und ET-18-OCH3 synergistisch um das 2,1-fache erhöht war. Da K562-Zellen klassische NK-Targetzellen sind, die keine HLA-Klasse I-Expression zeigen, ist die Zunahme der Lysis-Sensitivität gegen nicht-stimulierte Effektorzellen mit NK-Zellen korrelierbar. Eine Zugabe von IL-2 zu NK-Zellen verstärkt diese Aktivität. Trotzdem konnten Hsp70-spezifische NK-Zellen bislang aus allen getesteten Spendern (> 100) gewonnen werden. Dieser Befund konnte an mehr als 100 verschiedenen Spendern gezeigt werden. In allen Spendern konnte eine ruhende Hsp70-spezifische NK-Population nachgewiesen werden.

Obwohl die meisten Leukämiezellen auf Behandlungen mit zytostatischen und zytotoxischen Mitteln sensitiv reagieren, ist bekannt, daß einige Arten von Leukämiezellen wie K562-Zellen gegen toxische Wirkungen von ET-18-OCH3 sehr resistent sind. Erfindungsgemäß konnte gezeigt werden, daß eine kombinierte, aufeinanderfolgende Behandlung dieser Leukämiezellen mit Hitze und ET-18-OCH3 in subletalen Konzentrationen immunmodulatorische Aktivitäten zeigt, die mit einer erhöhten Translokation von Hsp70 an die Plasmamembran von K562-Zellen korrelierbar ist. Diese Zunahme an auf der Zelloberfläche exprimierten Hsp70-Molekülen weist einen signifikanten Einfluß auf die Sensitivität von K562-Tumorzellen gegenüber einer Lysis durch NK-Zellen auf. Zusätzlich dazu konnte die Aktivität von NK-Zellen durch Hsp70 auf der Membran von Tumorzellen gesteigert werden.

Es war zwar bekannt, daß bei einer Behandlung mit ET-18-OCH3 alleine eine Hsp70-Plasmamembranlokalisation ohne gleichzeitige Stimulation der Hsp70-Synthese induzierbar ist (5). Erfindungsgemäß konnte jedoch weiterhin gezeigt werden, daß Tumorzellen, insbesondere Leukämiezellen, die ansonsten gegen Behandlungen mit Etherlipiden hoch-resistent sind, durch eine kombinierte Behandlung mit einem Hitzeschock und einer subletalen Konzentration an ET-18-OCH3 durch NK-Zellen signifikant stärker lysierbar sind. Diese erhöhte Sensitivität für die Lysis durch NK-Zellen konnte mit einer Zunahme der Expression von Hsp70 an der Zelloberfläche korreliert werden.

Die erfindungsgemäß durchgeführten Untersuchungen zeigen somit, daß eine Kombination aus einer Hitzebehandlung mit Verabreichung einer subletalen Konzentration z.B. eines Etherlipids, beispielsweise ET-18-OCH3, die Sensitivität der Zielzellen, z.B. von Tumorzellen, bevorzugt von resistenten Leukämiezellen, beispielsweise von K562-Zellen, für die Lysis durch NK-Zellen deutlich erhöht. Diese Erkenntnisse können auch im klinischen Bereich eingesetzt werden. Da normale PBL-Zellen oder CD34-positive Vorläuferzellen auf ihrer Plasmamembran Hsp70 nicht exprimieren, und zwar weder unter physiologischen Bedingungen noch nach einer Hitzebehandlung und/oder einer Behandlung mit einem Etherlipid, ist ein derartiger klinischer Einsatz besonders angebracht, da keine Gefahr einer Autoimmunreaktion besteht. Gesunde Zelle werden nicht lysiert.

Es konnte gezeigt werden, daß die Wirksamkeit einer Purging-Behandlung bei der autologen Knochenmarkstransplantation bei Patienten mit einer akuten myeloischen Leukämie mit verbesserten, erkrankungsfreien Überlebensraten in vivo korreliert (6). Aufgrund der erfindungsgemäß durchgeführten Untersuchungen ist es nunmehr möglich, die Purging-Behandlung mit einer Hitzebehandlung und einer gleichzeitigen Behandlung in einer subletalen Konzentration eines Alkyl-Lysophospholipids, beispielsweise ET-18-OCH3, zu kombinieren. Durch die erhöhte Sensitivität der Leukämiezellen, die ansonsten gegenüber einer Behandlung mit einem Etherlipid als ausschließlichem Purging-Mittel resistent sind, gegenüber einer Lysis durch NK-Zellen, kann das Purging von Knochenmark deutlich verbessert werden. Weiterhin kann durch die erfindungsgemäß vorgeschlagene Kombinationsbehandlung von buffy-coat-Zellen (PBMC- oder Knochenmarkszellen) aus Leukämiepatienten, aber auch aus Patienten mit soliden Tumoren, die Zelloberflächenexpression von Hsp70 auf hochresistenten Leukämiezellen oder metastasierenden Zellen erhöht werden, so daß sie durch NK-Zellen mit Spezifität gegenüber Hsp70-exprimierenden Tumorzellen leichter lysierbar sind. Außerdem wird durch das in der Plasmamembran exprimierte Hsp70 die zytolytische Aktivität von NK-Zellen erhöht.

Nachfolgend werden die bei den erfindungsgemäßen Experimenten verwendeten Materialien und Methoden näher beschrieben. Sie sind jedoch lediglich als beispielhaft zu verstehen, und es stehen auch andere Materialien und Methoden zur Verfügung, um das erfindungsgemäße Verfahren auszuführen.

### ZELLEN und KULTURBEDINGUNGEN:

Die humane, chronisch myeloische Leukämiezellinie K562 wurde in RPMI, 1640 (Gibco, Eggenstein, Deutschland)- Medium kultiviert, das mit 10% hitzeinaktiviertem, fötalem Kälberserum (Gibco, Eggenstein, Deutschland) konditioniert war und 6 mM/l L-Glutamin und Penicillin (100 IU/ml)/Streptomycin (100 µg/ml) als Antibiotika enthielt. Die Zellen wurden in der exponentiellen Wachstumsphase bei einer Konzentration von 1-2 x 10⁶ Zellen pro ml Medium gehalten.

### PRÄPARATIONEN PERIPHERER BLUTLYMPHOZYTEN (PBL) und PROGENITORZELLEN:

Peripheres Blut und Nabelschnurblut wurden in sterilen Röhrchen gesammelt. Nach einer Anti-Koagulation mit Heparin (Heparin Novo, Novo Nordisk Pharma GmbH, Mainz, Deutschland) wurden periphere, mononukleäre Blutzellen (PBMC) und Nabelschnur-Lymphozyten durch Ficoll Isopaque (Ficoll Paque; Pharmacia, Uppsala, Schweden) in einer Dichtegradientenzentrifugation abgetrennt und in RPMI 1640-Medium, mit 10% FKS bei 37°C über Nacht inkubiert. PBL der Spender A, B und C wurden ebenfalls als Effektorzellen in den Zytotoxizitätstest verwendet. Als Targetzellen wurden PBL der Spender C, D und E und CD34-angereicherte Progenitorzellen aus Nabelschnurblut im gleichen Medium gehalten und, wie unten beschrieben, behandelt. Eine Anreicherung von CD34-Zellen wurde durch Zellsortierung in einem FACSStar^{-Plus}-Gerät (Becton Dickinson, Heidelberg, Deutschland) erhalten. Der Prozentsatz an CD34-positiv gefärbten Zellen wurde durch eine FACScan-Analyse, wie unten beschrieben, bestimmt.

### BILDUNG ADHÄRENTER NK-EFFEKTORZELLEN:

Natürliche Killerzellen wurden aus PBL aus Buffy-Coats, die aus gesunden, menschlichen Spendern stammten, durch Ficoll Isopaque (Pharmacia, Freiburg, Deutschland)-Dichtegradientenzentrifugation wie in (6) beschrieben, gewonnen. Kurz gesagt, wurden PBL (3-5 x 10⁸) in eine nicht-adhärente CD3+ T-Zellfraktion und eine adhärente NK-angereicherte Zellfraktion durch eine 12-stündige Inkubation in Kunststoff-Gewebekulturflaschen mit RPMI 1640-Medium, das rekombinantes IL-2 (100 IU/ml; Chiron, Frankfurt, Deutschland) enthielt, gewonnen. Die NK-angereicherte Zellfraktion wurde phänotypisch durch eine Durchflußzytometrieanalyse unter Verwendung von anti-CD3/CD16+CD56 und CD14/45 doppelt gefärbte Antikörpern (Becton Dickinson, Heidelberg, Deutschland) charakterisiert. Eine quantitative Durchflußzytometrieanalyse wurde unter Verwendung eines fluoreszenzaktivierten Zellsorters (FACScan mit PC Lysis II Software, Becton Dickinson, Heidelberg, Deutschland) durchgeführt. Eine NK-angereicherte Effektorzellpopulation wurde für Zytotoxizitätsstudien am Tag 4 nach der Auftrennung verwendet; der Prozentsatz der für die Zytotoxizitätsexperimente verwendeten NK-Zellen lag bei 40% bis 60%.

### HITZEBEHANDLUNG und BEHANDLUNG mit ALKYL-LYSOPHOSPHOLIPID:

Exponentiell wachsende K562-Zellen, CD34-angereicherte Progenitorzellen und PBL wurden bei der nicht-letalen Temperatur von 41,8°C 2 Stunden lang in einem Temperatur-kontrollierten Wasserbad (Haake E3, Karlsuhe, FRG) behandelt und dann bei 37°C 14 Stunden lang inkubiert. 1-Octadecyl-methyl-rac-glycero-3-phosphocholin (ET-18-OCH3, Edelfosin) wurde frisch in einer 500 µg/ml Stammlösung in RPMI 1640-Medium durch Erhitzen auf 50°C für 30 Minuten und 3-maliger Ultraschallbehandlung gelöst. Nicht-behandelte oder mit Hitze behandelte Zellen (1 x 10⁶ Zellen/ml) wurden mit der nicht-toxischen Konzentration von 25 µg/ml 2 Stunden lang bei 37°C inkubiert. Nach dieser Behandlung wurden die Zellen 3x in phosphatgepufferter Salzlösung (PBS) gewaschen und dann in frischem Kulturmedium ohne ET-18-OCH3 weitere 2 Stunden lang bei 37°C inkubiert.

Die nicht-letale Temperatur von 41,8°C und die nicht-toxische ET-18-OCH3-Konzentration wurden durch Überlebensfähigkeitstests unter Verwendung von Trypanblau und Propidiumiodid und durch Apoptosemessungen bestimmt (vgl. (5)).

Das Detergens Polyoxyethylensorbat 20 (Tween20; Sigma, München, Deutschland) wurde als membraninteraktives Kontrollmittel bei der nicht-letalen Konzentration von 5 ppm eingesetzt. Eine Tween20-Behandlung der Zellen wurde unter den identischen Bedingungen durchgeführt, wie sie für ET-18-OCH3 beschrieben wurde.

### MEMBRANPRÄPARATION und ZYTOPLASMAFRAKTIONEN:

Die Plasmamembranpräparation wurde entsprechend einem leicht modifizierten Verfahren nach Bauer und Hurtenbach (7) wie vorher beschrieben (6) durchgeführt. Kurz gesagt wurden 50 x 10⁶ Zellen, die entweder unbehandelt oder behandelt waren, in eiskaltem Puffer (1 mM Tris-HCL, 5mH MgCl₂, pH 7,4) gespült. Das Zellpellet wurde in eiskalter 3 mM phosphatgepufferter Sucroselösung (0,32 M, pH 7,6, 1 mM EDTA) resuspendiert und in einem Dounce-Homogenisator durch 50 Stöße mit einem eng anliegenden Pistill homongenisiert. Nach Zentrifugation (2000 g, 4°C für 15 Minuten) wurde das Pellet rehomogenisiert. Die vereinigten Überstände wurden bei 11000 g bei 4°C 25 Minuten lang abzentrifugiert. Der erhaltene Überstand wurde bei 20000 g bei 4°C 45 Minuten lang zentrifugiert. Das letzte Pellet enthielt Membrane und membrangebundene Proteine und wurde in 100 µl PBS resuspendiert.

Zur Gewinnung der Zytoplasmaproteinfraktionen wurden 5 x 10⁶ Zellen in 10 mM trisgepufferter Saline (pH 7,5), enthaltend 1% Nonidet P-40 (NP-40, Sigma) und 1 mM Phenylmethylsulphonylfluorid (PMSF, Sigma), 45 Minuten lang auf Eis, verdünnt in Laemmlis Probenpuffer, lysiert.

### SDS-PAGE und WESTERN-BLOT-ANALYSE:

Gleiche Proteinmengen (10 µg) wurden auf einem 10% SDS-PAGE wie in (5) beschrieben gemäß dem Verfahren von Laemmli (8) einer Elektrophorese unterzogen. Nach der SDS-PAGE-Elektrophorese wurden die Proteine auf Immobilon PVDF-Membrane (Millipore Corp., Bedford, MA) übertragen, wobei ein Standardprotokoll (9) angewandt wurde. Eine nicht-spezifische Bindung der Membran wurde mit 5% Magermilch in PBS blockiert. Die Blots wurden mit primärem Hsp70-Antikörper (Amersham, Braunschweig, Deutschland) und dem sekundären Antikörper (Ziege-anti-Maus-IgG-Peroxidasekonjugiert, BioRad, Deutschland) je 1 Stunde lang inkubiert. Die Immunkomplexe wurden unter Verwendung des ECL-Detektionssystems (Amersham, Braunschweig, Deutschland) nachgewiesen.

Die Autroradiografien wurden durch Laserdensitometrie (Ultrascan XL, Pharmacia, Freiburg, Deutschland) quantifiziert. Die relativen Hsp70-Mengen wurden durch das Verhältnis der integrierten Fläche unter jedem Peak zu den unbehandelten Zellen, die als interner Standard verwendet wurden, bestimmt.

### ZYTOTOXIZITÄTSTEST (CML):

Der Zytotoxizitätstest der nicht-stimulierten PBL und der NK-angereicherten Effektorzellen wurde in einem 4-stündigen Chrom-51-Standardfreisetzungstest (6) durchgeführt.

Die Targetzellen (K562-Zellen, PBL oder CD34-angereicherte Progenitorzellen) wurden mit 0,01mCi/ml Natrium Cr-51 2 Stunden lang inkubiert. Nach zwei Waschschritten wurde die Targetzellzahl bestimmt, und der CML-Test wurde in 96-Vertiefungen aufweisenden Mikrotiterplatten bei verschiedenen Effektor-zu-Target-Verhältnissen im Bereich von 30:1 bis 3,5:1 NK-Zellen oder 60:1 bis 15:1 für nicht-stimulierte PBL als Effektorzellen durchgeführt. Der Prozentsatz der spezifischen Lysis wurde berechnet als: [(experimentelle Freisetzung - spontane Freisetzung) : (maximale Freisetzung - spontane Freisetzung)] x 100. Der Prozentsatz der spontanen Freisetzung wurde berechnet als: (spontane Freisetzung : maximale Freisetzung) x 100, und der Prozentsatz lag immer unter 15% für jede Targetzelle.

Die Antikörper-Blockierungsexperimente wurden unter Verwendung des Hsp70-spezifischen, monok lonalen Antikörpers RPN1197 (Amersham) in einer Konzentration von 1µg/1 x 10⁶ Zellen durchgeführt. Nach erfolgter Markierung wurden die Targetzellen mit dem Antikörper 1 Stunde lang inkubiert, und dann wurde der Zytotoxizitätstest wie oben beschrieben durchgeführt.

### STATISTISCHE ANALYSE:

Die Signifikanz der Unterschiede zwischen den verschiedenen, experimentellen Werten wurde durch den t-Test von Student bestimmt.

Bei der therapeutischen Anwendung werden 100 - 1000 Mio. Lymphozyten, bevorzugt 200 - 800 Mio. Lymphozyten, reinfundiert.

### LITERATUR

(1) Heesbeen EC., Rijksen G., van-Heugten HG., Verdonck LF.: Influence of serum levels on leukemic cell destruction by the ether lipid ET-18-OCH3. Leuk. Res. 19: 417, 1995
(2) Okamoto S., Olson AC., Berdel WE., Vogler WR.: Purging of acute myeloid leukemic cells by ether lipids and hyperthermia. Blood 72: 1777, 1988
(3) Fujiwara K., Modest EJ., Welander CE., Wallen CA.: Cytotoxic interactions of heat and an ether lipid analogue in human ovarian carcinoma cells. Cancer Res. 49: 6285, 1989
(4) Fujiwara K., Modest EJ., Wallen CA.: Cell kill and cytostasis by ET-18-OCH3 and heat. Anticancer Res. 15: 1333, 1995
(5) Botzler C., Kolb HJ., Issels RD., Multhoff G.: Noncytotoxic alkyl-lysophospholipid treatment increases sensitivity of leukemic K562 cells to lysis by natural killer (NK) cells. Int. J. Cancer 65: 633, 1996
(6) Multhoff G., Botzler C., Wiesnet M., Eissner G., Issels R.: CD3 large granular lymphocytes recognize a heat-inducible immunogenic determinant associated with the 72-kD heat shock protein on human sarcoma cells. Blood 86: 1374, 1995
(7) Bauer HC., Hurtenbach U.: Murine cortical brain cells are autoantigenic from a distinct developmental stage onwards. JN. 12: 1, 1986
(8) Laemmli UK.: Cleavage of structural proteins during the assembly of the head of bacteriophage T4. Nature 227: 680, 1970
(9) Towbin H., Staehelin T., Gordon J.: Electrophoretic transfer of proteins from polyacrylamide gels to nitrocellulose sheets: procedure and some applications. Proc. Natl. Acad. Sci. USA 76: 4350, 1979
(10) Berdel, W.E.: Membrane interactive lipids as experimental anticancer drugs, Brit. J. Cancer 64:208-211,1991

**TABELLE 1**

| BEHANDLUNG | ANTEIL ÜBERLEBENDER ZELLEN (%±SD) | | | BEHANDLUNG | ANTEIL ÜBERLEBENDER ZELLEN (%±SD) | |
|---|---|---|---|---|---|---|
| hs⁺ ET-18-OCH3 | K562 Zellen | PBL | CD34 Zellen | hs⁺ Tween20 | K562 Zellen | PBL |
| 100µg/mL | 2 ± 2 | 0 | 0 | 1000ppm | 3 ± 1 | 0 |
| 75µg/mL | 35 ± 7 | 0 | n.t. | 500ppm | 32 ± 5 | 16 ± 3 |
| 50µg/mL | 80 ± 5 | 90 ± 6 | n.t. | 10ppm | 69 ± 4 | 95 ± 2 |
| 25µg/mL | 97 ± 3 | 95 ± 4 | 88 ± 2 | 5ppm | 99 ± 1 | 98 ± 2 |
| 10µg/mL | 98 ± 2 | 98 ± 2 | 96 ± 5 | 1ppm | 97 ± 2 | 98 ± 2 |

**TABELLE 2**

| BEHANDLUNG | PBL | CD34 ZELLEN |
|---|---|---|
| unbehandelt | 1.0 ± 1.1 | 1.86 ± 1.4 |
| ET-18-OCH3(25µg) | 1.1 ± 0.7 | 1.30 ± 0.5 |
| Hitze (41.8°C) | 2.8 ± 1.2 | 1.37 ± 0.6 |
| Hitze und ET-18-OCH3 | 1.8 ± 0.9 | 1.73 ± 1.3 |

**TABELLE 3**

| BEHANDLUNG | LYSE-VERHÄLTNIS Mittelwerte±SD | p-WERTE |
|---|---|---|
| untr/Tween20 | 1.180 ± 0.241 | 0.271 |
| untr/ET-18-OCH3 | 1.341 ± 0.023* | 0.032 |
| untr/hs | 1.316 ± 0.026* | 0.006 |
| untr/ hs+Tween20 | 1.300 ± 0.122 | 0.124 |
| untr/hs+ET-18-OCH3 | 1.845 ± 0.214* | 0.004 |
| Hitze/hs+Tween20 | 1.001 ± 0.064 | 0.300 |
| Hitze/hs+ET-18-OCH3 | 1.32 ± 0.072* | 0.006 |

**TABELLE 4**

| BEHANDLUNG | PBL-LYSEVERHÄLTNIS Mittelwerte ± SD | CD34-ZELLLYSEVRHÄLTNIS Mittelwerte ± SD |
|---|---|---|
| untr/hs | 0.98 ± 0.21 | 1.18 ± 0.12 |
| untr/hs+ET-18-OCH3 | 1.01 ± 0.11 | 1.08 ± 0.20 |
| Hitze/hs+ ET-18-OCH3 | 1.09 ± 0.09 | 1.13 ± 0.15 |

## Patentansprüche

1. Verfahren zur Induktion einer durch NK-Zellen vermittelten Immunantwort, ex vivo,
**dadurch gekennzeichnet**, daß
eine zumindest Tumorzellen oder durch Viren, Bakterien und/oder Pilze infizierte tierische oder menschliche Zellen (Zielzellen) und NK-Zellen enthaltende physiologische Zellsuspension mit den nachfolgenden Verfahrensschritten in der angegebenen Reihenfolge behandelt wird, um die Sensitivität der Zielzellen für die Lyse durch NK-Zellen zu erhöhen:
a) Hitzebehandeln der in der Suspension enthaltenen Zellen bei einer Temperatur von 38°C bis 43°C für eine Zeit von mindestens 1 Stunde;
b) Absenken der Temperatur auf physiologische Zelltemperatur (etwa 37°C), um den in der Suspension enthaltenen Zellen eine Erholungsphase von mindestens 1 Stunde zu gewähren;
c) Zugabe einer den membrangebundenen Hsp70-Anteil der Zielzellen erhöhenden Verbindung in einer für die Zellen subletalen Konzentration und Einwirkenlassen dieser Verbindung für mindestens 30 Minuten;
d) Erholungsphase von mindestens 1 Stunde bei etwa 37°C.

2. Verfahren nach Anspruch 1,
dadurch gekennzeichnet, daß
als Tumorzellen Leukämiezellen oder Tumorzellen aus Metastasen solider Tumoren oder durch HIV infizierte Zellen oder durch Mycobakterien infizierte Zellen verwendet werden.

3. Verfahren nach Anspruch 2,
dadurch gekennzeichnet, daß
als Leukämiezellen K562-Zellen eingesetzt werden.

4. Verfahren nach Anspruch 2,
dadurch gekennzeichnet, daß
als Tumorzellen metastasierte Zellen solider Tumore aus Karzinomen, Sarkomen, Melanomen oder Lymphomzellen eingesetzt werden.

5. Verfahren nach einem oder mehreren der vorhergehenden Ansprüche,
dadurch gekennzeichnet, daß
als Verbindung ein Alkyl-Lysophospolipid eingesetzt wird.

6. Verfahren nach Anspruch 5,
dadurch gekennzeichnet, daß
als Alkyl-Lysophospolipid ET-18-OCH3 (Edelfosin) eingesetzt wird.

7. Verfahren nach einem oder mehreren der vorhergehenden Ansprüche,
dadurch gekennzeichnet, daß
im Verfahrensschritt (a) eine Temperatur im Bereich von 39°C bis 42°C eingesetzt wird.

8. Verfahren nach Anspruch 7,
dadurch gekennzeichnet, daß
eine Temperatur im Bereich von 41°C bis 42°C eingesetzt wird.

9. Verfahren nach einem oder mehreren der vorhergehenden Ansprüche,
dadurch gekennzeichnet, daß
das Alkyl-Lysophospholipid in einer Konzentration von 1 - 30 µg/ml eingesetzt wird.

10. Verfahren nach Anspruch 9,
dadurch gekennzeichnet, daß
das Alkyl-Lysophospholipid in einer Konzentration von 10 - 25 µg/ml, bevorzugt 15-20 µg/ml eingesetzt wird.

11. Verfahren nach einem oder mehreren der vorhergehenden Ansprüche,
dadurch gekennzeichnet, daß
die Hitzebehandlung für einen Zeitraum von 1 bis 3 Stunden erfolgt.

12. Verfahren nach Anspruch 11,
dadurch gekennzeichnet, daß
die Hitzebehandlung für einen Zeitraum von 2 bis 3 Stunden erfolgt.

13. Verfahren nach einem oder mehreren der vorhergehenden Ansprüche, dadurch gekennzeichnet, daß die Behandlung mit dem Alkyl-Lysophospholipid für einen Zeitraum von 30 Minuten bis 3 Stunden erfolgt.

14. Verfahren nach Anspruch 13,
dadurch gekennzeichnet, daß
die Behandlung mit dem Alkyl-Lysophospholipid für einen Zeitraum von einer Stunde bis zwei Stunden erfolgt.

15. Verfahren nach einem oder mehreren der vorhergehenden Ansprüche,
dadurch gekennzeichnet, daß
NK-Zellen eingesetzt werden, die zumindest CD16 exprimieren und/oder durch IL-2 stimulierbar sind und/oder kein CD3 exprimieren und/oder keine α/β- oder γ/δ-T-Zell-Rezeptoren aufweisen und/oder nicht vom MHC-Typ des Patienten abhängig sind.

16. Verfahren nach einem oder mehreren der vorhergehenden Ansprüche,
dadurch gekennzeichnet, daß
die Erholungsphase im Schritt (b) und (d) für einen Zeitraum von 2 bis 16 Stunden durchgeführt wird.

17. Verfahren nach Anspruch 16,
dadurch gekennzeichnet, daß
die Erholungsphase für einen Zeitraum von 8 bis 12 Stunden durchgeführt wird.

18. Verfahren nach einem oder mehreren der vorhergehenden Ansprüche,
dadurch gekennzeichnet, daß
als zumindest Zielzellen und NK-Zellen enthaltende physiologische Zellsuspension Vollblut und/oder Buffy-Coat-Zellen oder eine zumindest Zielzellen und NK-Zellen enthaltende Fraktion hiervon eingesetzt wird.

19. Verwendung der nach einem Verfahren nach einem oder mehreren der vorhergehenden Ansprüche behandelten Zellsuspension zur Reinfundierung in den Patienten.

20. Verwendung nach Anspruch 19,
dadurch gekennzeichnet, daß
die Zellsuspension zur Reinfundierung in den Patienten zur Behandlung von Tumorerkrankungen oder durch Viren, Bakterien und/oder Pilze hervorgerufene Erkrankungen eingesetzt wird.

21. Verwendung eines Verfahrens nach einem oder mehreren der vorhergehenden Ansprüche zur Steigerung der Expression von Hsp70-Protein auf der Zellmembran der Zielzellen.

22. Verwendung eines Verfahrens nach einem oder mehreren der vorhergehenden Ansprüche zur Behandlung eines Patienten mit einer Tumorerkrankung oder einer durch Viren, Bakterien und/oder Pilze hervorgerufenen Erkrankung.
